# EUROPEAN PATENT APPLICATION

(11) **EP 1 772 066 A1**
(43) Date of publication of application: **11.04.2007**
(21) Application number: 05730522.9
(22) Date of filing: 14.04.2005
(51) Int. Cl.: A23L 1/30

(54) **HEALTH FOOD, PROCESS FOR PRODUCING THE SAME AND DRIED MATTER OR EXTRACT**

(30) Priority: 14.04.2004 JP 2004118539
(71) Applicant: Keio University, Tokyo 108-8345 (JP)
(72) Inventor: UMEZAWA, Kazuo; c/o Keio University Faculty of, Kohoku-ku,Yokohama-shi,Kanagawa;2238522 (JP); TAKEI, Izumi; c/o Keio University School of, Shinjuku-ku, Tokyo;1608582 (JP); SHIBAHARA, Seiji, 1940001 (JP); KOJIMA, Itaru, 3710801 (JP); KOYANO, Takashi, 1640012 (JP); ISHII, Chika; c/o Keio University Faculty of, Kohoku-ku,Yokohama-shi,Kanagawa;2238522 (JP)
(74) Representative: Horner, Martin Grenville
(86) International application number: PCT/JP2005/007239
(87) International publication number: WO 2005/099485

(57) **Abstract**

Conophylline, contained in leaves of Ervatamia microphylla or Tabernaemontana divaricata, can induce non-tumor cells to differentiate into insulin-producing cells and thereby to produce insulin and also to lower blood glucose levels. Thus, in the present invention, dried products and extracts of leaves of Ervatamia microphylla or Tabernaemontana divaricata, which contain conophylline, are used as health foods for preventing and improving diabetes and obesity or health foods for lowering blood glucose levels.

## Description

### CROSS REFERENCE TO RELATED APPLICATIONS

This application claims the benefit of priority to Japanese Patent Application No. 2004-118539, filed on April 14, 2004, which is incorporated herein by reference.

### TECHNICAL FIELD

The present invention relates to health foods, methods for producing the same, and dried products or extracts of leaves of Ervatamia microphylla or Tabernaemontana divaricata to be used for such foods and methods.

### BACKGROUND ART

In recent years, for the purpose of alternative medicine, there has been a growing need for various herbs, traditional Chinese medicine, and health food derived from flowers, leaves, stems, roots, peels, etc. of plants. This is because, in this age of gluttony when people have been substantially satisfied with "nutrition" and "delicacy," their concern is shifting to prevention of lifestyle-related diseases etc. by improving foods and/or diet.

In particular, in terms of prevention of disease, health foods such as supplements (dietary supplement) and foods for specified health uses, etc. can be ingested for a longer term and are more easily accepted to the general people than drugs because of their fewer side effects and higher safety. Further, patients suffering from complex diseases have a problem of riskful drug overlap; there has been a demand for use of health foods as alternatives to part of or all the regularly used drugs.

Among health foods, new foods that contribute in particular to disease prevention by regulating physiological functions are referred to as functional foods. Unlike drugs, such health foods are designed to be taken mainly by healthy people for a long term as means of preventing diseases and controlling bodily functions.

Meanwhile, leaves of Ervatamia microphylla, an Apocynaceae family plant, contains conophylline, which is known to be useful as an anti-tumor agent (for example, refer to Drugs Exptl. Clin. Res. 22, 35-40, 1996). It is also known that conophylline induces differentiation of pancreatic acinar carcinoma AR42J-B13 cells into insulin-producing cells. (The Book of Abstracts (01-21) of the 45th Annual Meeting of the Japan Diabetes Society held in Tokyo, published on May 18, 2002). Under this condition, however, the insulin-producing cells that have been induced do not release insulin from the cells.

Moreover, it has not been clarified whether conophylline lowers blood glucose levels in animals.

### DISCLOSURE OF THE INVENTION

### PROBLEMS TO BE SOLVED BY THE INVENTION

Thus, the object of the present invention is to provide dried products or extracts of leaves of Ervatamia microphylla or Tabernaemontana divaricata; health foods containing as an effective ingredient leaves of Ervatamia microphylla or Tabernaemontana divaricata, dried products of the leaves, or extracts of the leaves or of dried products of the leaves; and methods for producing the same, for preventing or improving diabetes.

### SUMMARY OF THE INVENTION

The inventors found that, by administering to streptozotocin-induced diabetic rats conophylline (about 500 mg) obtained by isolating and purifying the oily substance (about 130 g) by silica gel column chromatography, which was extracted with 100 L of chloroform from 4 kg of Ervatamia microphylla leaves, their blood glucose levels are lowered. They also found that an extract of leaves of Ervatamia microphylla and an extract of leaves of crape jasmine (the Tabernaemontana divaricata cultivated in Japan) exhibits the same drug efficacy as that of conophylline. This suggests that extracts of these leaves do not contain any inhibitor that inhibits the function of conophylline. It was further found that Ervatamia microphylla leaves have no cytotoxicity as long as they are used at a low concentration. The present invention has thus been accomplished. It should be noted that Ervatamia and Tabernaemontana are known to be in the same genera (refer to J. of Ethnopharmacology 10, 1-156, 1984). The present invention takes the most advantage of fact that conophylline has been obtained from nature.

Thus, the health food for preventing or improving diabetes according to the present invention contains as an effective ingredient an Ervatamia microphylla leaf, a dried product of the leaf, or an extract of the leaf or of the dried product of the leaf. It should be noted that the aforementioned health food bears a note stating that the food is intended for use to prevent and improve diabetes.

Alternatively, the health food for lowering blood glucose levels according to the present invention contains as an effective ingredient an Ervatamia microphylla leaf, a dried product of the leaf, or an extract of the leaf or of the dried product of the leaf. It should be noted that the aforementioned health food bears a note stating that the food is intended for use to lower blood glucose levels.

The health food according to the present invention may further contain hepatocyte growth factor, and may contain nicotinamide.

The method for producing a health food for preventing or improving diabetes according to the present invention uses as a basic ingredient a leaf of Ervatamia microphylla or Tabernaemontana divaricata, a dried product of the leaf, or an extract of the leaf or of the dried product of the leaf.

The method for producing a health food for lowering blood glucose levels according to the present invention uses as a basic ingredient a leaf of Ervatamia microphylla or Tabernaemontana divaricata, a dried product of the leaf, or an extract of the leaf or of the dried product of the leaf.

Further, the scope of present invention includes a dried product of a leaf of Ervatamia microphylla or Tabernaemontana divaricata or an extract of the leaf or of a dried product of the leaf.

It should be noted that the aforementioned dried product includes products obtained by directly drying a leaf of Ervatamia microphylla or Tabernaemontana divaricata, products obtained by drying the leaf after pulverization, and products obtained by pulverizing the leaf after direct drying.

In addition, the aforementioned extract refers to an aqueous extract (from which debris of the leaf may or may not be removed, and which may be concentrated or diluted) obtained by applying a solvent to a leaf of Ervatamia microphylla or Tabernaemontana divaricata or its dried product and extracting the effective ingredient (conophylline in particular) contained in the leaf or its dried product into the solvent, or a solid obtained by removing the solvent in the aqueous extract.

### BRIEF DESCRIPTION OF THE DRAWINGS

FIG. 1 shows the effect of Ervatamia microphylla leaves on AR42J cells in one example according to the present invention.
FIG. 2 shows the effect of conophylline on blood glucose levels of streptozotocin-administered rats in one example according to the present invention.
FIG. 3 shows the results of examination of cytotoxicity in each of the five fractions obtained by silica gel column chromatography from a methanol extract of Ervatamia microphylla leaves in one example according to the present invention. "O," "□," "X," "Δ," and "*" in the figure show the result of fraction 1, fraction 2, fraction 3, fraction 4, and fraction 5, respectively.
FIG. 4 shows the effect of a methanol extract of leaves of Ervatamia microphylla or crape jasmine (the Tabernaemontana divaricata cultivated in Japan) on K-ras-NRK cells in one example according to the present invention.

### BEST MODE FOR CARRYING OUT THE INVENTION

As mentioned above, conophylline contained in Ervatamia microphylla leaves exhibits the effect of lowering the blood glucose level of diabetes-induced rats and the extract of Ervatamia microphylla leaves has the same efficacy as that of conophylline. Thus health foods containing as an effective ingredient conophylline-containing leaves of Ervatamia microphylla, dried products of the leaves, or extracts of the leaves or of the dried products of the leaves are considered to be able to lower the blood glucose level. Likewise, since the extract of leaves of crape jasmine (the Tabernaemontana divaricata cultivated in Japan) has the same drug efficacy as conophylline, health foods containing as an effective ingredient conophylline-containing leaves of crape jasmine (the Tabernaemontana divaricata cultivated in Japan), dried products of the leaves, or extracts of the leaves or of the dried products of the leaves are considered to be able to lower the blood glucose level. Therefore, health foods containing as an effective ingredient conophylline-containing leaves of Ervatamia microphylla or Tabernaemontana divaricata, dried products of the leaves, or extracts of the leaves or of the dried products of the leaves are considered to be useful in preventing or improving diabetes (type 2 diabetes in particular) or obesity. Generally, it is particularly difficult to offer guidance on dietary restrictions and exercise to people who do not need to be treated with drugs but are very likely to develop diabetes or obesity if left uncared. However, by providing such people with health foods containing as an effective ingredient conophylline-containing leaves of Ervatamia microphylla or Tabernaemontana divaricata, dried products of the leaves, or extracts of the leaves or of the dried products of the leaves, it becomes possible to prevent or improve their diabetes or obesity without imposing dietary restrictions or exercise.

Further, by using conophylline-containing leaves of Ervatamia microphylla or Tabernaemontana divaricata, dried products of the leaves, or extracts of the leaves or of the dried products of the leaves as health foods, it becomes possible to make effective use of conophylline, whose production process from leaves of Ervatamia microphylla or Tabernaemontana divaricata is considered to be complicated and whose purification is not effective.

### == Production Example of Health Foods ==

Dried products of leaves of Ervatamia microphylla or Tabernaemontana divaricata can be obtained by drying leaves of Ervatamia microphylla or Tabernaemontana divaricata or pulverized leaves of Ervatamia microphylla or Tabernaemontana divaricata by procedures such as air drying, freeze drying, warm air drying, etc. Dry powder of leaves of Ervatamia microphylla or Tabernaemontana divaricata can be obtained by pulverizing dried products of these leaves.

On the other hand, an aqueous extract of leaves of Ervatamia microphylla or Tabernaemontana divaricata can be obtained by applying a solvent to leaves of Ervatamia microphylla or Tabernaemontana divaricata or their dried products and extracting the active ingredients contained in them into the solvent. As the solvent, for example, water, alcohol, ethyl acetate, chloroform, or a mixed solvent of two or more of these can be used. From the viewpoint of safety, it is preferable to use water, ethanol, or their mixture. It should be noted that in extraction of the active ingredients into a solvent, by performing a secondary action such as stirring, crushing, heating, or the like, it is possible to increase the amount of the active ingredients in the extract and to shorten their extraction time. In this embodiment, an aqueous extract of leaves of Ervatamia microphylla or Tabernaemontana divaricata is used directly as a health food without removing the leaf debris, but an aqueous extract in which the leaf debris has been removed by filtration or centrifugation can be used.

Furthermore, a solid can be obtained by vaporing or drying of the aqueous extract to remove the solvent, into which the active ingredient has been extracted. It should be noted that, for drying of extracts, air drying, freeze drying, warm air drying, or the like can be used.

The usage forms of the health food according to the present invention are, for example, extracts (e.g., tea) obtained by submerging dry leaves in hot water; solutions (e.g., green juice) obtained by pulverizing in a blender after adding water; decoctions (e.g., traditional Chinese medicine) obtained by decocting dry leaves; supplement in the forms of capsules, tablets, granules, etc; chewing gums; hard sweets (e.g., candies, drops, etc.); seasoned powders for sprinkling over rice, etc, but are not limited to them.

The health food according to the present invention contains as an effective ingredient a leave of Ervatamia microphylla or Tabernaemontana divaricata, a dried product of leaves, an extract of the leaves or of the dried product of the leaves. Other substances, such as hepatocyte growth factor (HGF), nicotinamide, or the like, which induce pancreatic endocrine precursor cells to differentiate into pancreatic endocrine cells may be contained. Beside these, substances that do not hamper preventing or improving of diabetes or obesity and lowering of blood glucose levels, particularly such as natural products or substances derived from them, are preferred from the viewpoint of safety. Examples of such substances include deoxynojirimycin, zinc, magnesium, chromium, chromium picolinate, antioxidants, etc. Deoxynojirimycin, contained in large amounts in mulberry leaves and tea leaves, can suppress the action of α-glucosidase, which breaks starch and sucrose into glucose, suppress the uptake of glucose from the small intestine and suppress an increase in postprandial blood glucose levels. Zinc can help insulin synthesis and secretion in pancreatic cells, and magnesium, chromium, chromiumpicolinate, etc. can enhance the sensibility of insulin, and can activate the action of insulin. Examples of antioxidants to be used include vitamin E, vitamin C, β-carotene, polyphenol, etc.

It should be noted that the health food according to the present invention can be in any form such as solid, powder, liquid, fluid, cream, gel, etc. In addition, the health food according to the present invention is designed in principle for healthy people, but may be digested by those patients suffering from a disease for which the health food is effective.

### EXAMPLES

Hereinafter, the present invention will be explained in more detail with reference to Examples and drawings.

### [EXAMPLE 1]

An oily substance (about 130 g), extracted with 100 L of chloroform from 4 kg of dried product obtained by drying Ervatamia microphylla leaves (harvested in Khon Khen, Thailand) at 60°C for 2 hours, was fractionated by a silica gel column chromatography; that is, the substance was passed five times through about 500g of silica gel packed into a column and the adsorbate was eluted sequentially with eluents of chloroform: methanol at a ratio of 40: 1 and 20: 1. Then, the active fractions that induce a morphological change in K-Ras-NRK cells were recovered. Next, conophylline (about 500 mg) was purified by separating and concentrating these recovered active fractions by chromatography using a silica gel column (about 500 g of silica gel) with an eluent of n-hexane:ethyl acetate at a ratio of 1:2 and an eluent of ethyl acetate and a silica gel column (about 150 g of silica gel) with eluents of n-hexane:ethyl acetate:chloroform at ratios of 9:3:1 and 6:3:1..

### EXAMPLE 2

The inventors have found that, by culturing AR42J cells (ATCC CRL 1492) in a culture media supplemented with conophylline, the cells are induced to differentiate into insulin-producing cells. Thus, it was examined whether an extract of Ervatamia microphylla can induce AR42J cells to differentiate into insulin-producing cells. It should be noted that, since characteristic morphological changes of cells are observed as differentiation into insulin-producing cells is induced, morphological changes in AR42J cell were examined as a marker of differentiation induction in this Example. AR42J cells were cultured in the media prepared by sterilizing Dulbecco modification Eagle' medium (DMEM; Nissui Pharmaceutical Co., Ltd.) by filtration after its pH was adjusted to 7.4 in the presence of 20 mM HEPES-NaOH and adding 0.6 6 g/l glutamine (Kanto Chemical Co., Inc: Tokyo, Japan), 0.2 g/l kanamycin (Sigma Chemical Science; Sigma:St.Louis, MO, USA), 100 unit/ml penicillin G (Sigma), 5 mM NaHCO₃, and 10% fetal bovine serum (FBS; Sigma).

500 µl of a suspension of AR42J cells (4.0 x 10⁵ cells/ml per culture medium) was plated into each well of 24-well plastic plates (Corning) at 2 x 10⁵ cells/well and cultured under the conditions of 37°C and 5% CO₂. The next day, a solution (final concentration: 0.1 µg (corresponding to 800 µg of dried product of Ervatamia microphylla leaves)/ml) prepared by diluting conophylline purified in Example 1 with methanol and a suspension (final concentration: 30 µg/ml) of a dried product of Ervatamia microphylla leaves were supplemented to the media and the plates were incubated at 37°C under 5% CO₂ for three days. It should be noted that a suspension (pasty fluid) of a dried product of Ervatamia microphylla leaves was prepared by applying 60 ml of water to 4. 01 g of a dried product of Ervatamia microphylla leaves and grinding the mixture in a food mill (Millser; Iwatani International Corp., Tokyo, Japan).

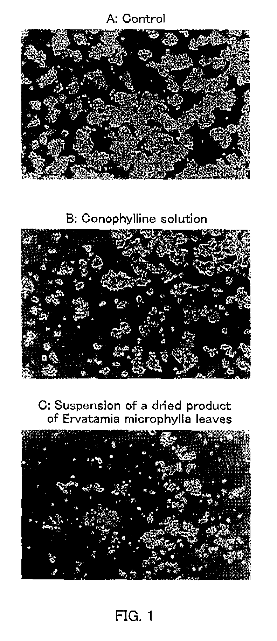

After three days, the culture was transferred to an Eppendorf tube. The cells were washed once in 200 µl of PBS⁻ (Ca²⁺ and Mg²⁺-free PBS; 8.0 g/1 NaCl, 0.2 g/l KCl, 0.916 g/l Na₂HPO₄, 0.2 g/1 KH₂PO₄) and then 200 µl of fresh PBS⁻ was added. The morphology of the viable cells was observed and the cells were photographed at 150X magnification with a camera linked to the microscope. The cells cultured in the medium without addition of conophylline nor an extract of Ervatamia microphylla leaves were used as the control. The results are shown in FIG.1 (control: FIG. 1A; conophylline solution: FIG. 1B; suspension of a dried product of Ervatamia microphylla leaves: FTG.1C).

As shown in FIG. 1, it was found that, the AR42J cells supplemented with a suspension of a dried product of Ervatamia microphylla leaves, like cells supplemented with conophylline solution, undergo a morphological change from small spherical cells to flat cells partly with processes, i.e., be induced to differentiate into insulin-producing cells. This indicates that, by using Ervatamia microphylla leaves, cells can be induced to differentiate into insulin-producing cells without the need of purifying conophylline.

Further, it was found that, the direct use of Ervatamia microphylla leaves requires a smaller amount of leaves for induction of differentiation into insulin-producing cells than the use of purified conophylline from Ervatamia microphylla leaves. Thus, it was revealed that induction of differentiation into insulin-producing cells is not inhibited even when using Ervatamia microphylla leaves, instead of purifying conophylline.

### EXAMPLE 3

Ten 1-day-old Wistar rats (purchased from Japan SLC, Shizuoka, Japan) were intraperitoneally injected with streptozotocin (purchased from Wako Pure Chemical Industries, Ltd., dissolved in 0.05 mM citrate buffer (pH 4.5)) at 85 µg/g BW per rat. Streptozotocin decreases insulin production by destroying pancreatic cells, thereby inducing diabetes. The day of streptozotocin injection was defined as day 0. Starting from the next day (day 1), five rats were injected subcutaneously with a solution (ethanol) of conophylline at 5 µg/g BW for seven consecutive days and their blood glucose levels were measured at a predetermined time daily. Five rats in the control group received the same volume of solvent (control). As a result, as shown in FIG. 4, all the rats showed a remarkable increase in the blood glucose level when streptozotocin was administered, whereas the conophylline (·)-administered rats showed an apparent decrease in blood glucose levels, compared with the control (o), indicating the effect of lowering the blood glucose level by conophylline (*: P < 0.05, **: P < 0.01 vs Control).

The blood glucose level-lowering effect of conophylline was thus confirmed in rats. Therefore, it is considered that even when using conophylline-containing Ervatamia microphylla leaves or their dried products, the blood glucose level can be lowered in animals. In this case, it is considered that the direct use of conophylline-containing Ervatamia microphylla leaves or their dried products lowers the blood glucose level in a smaller amount of leaves.

### EXAMPLE 4

Next, in consideration of using conophylline-containing leaves and extracts for foods and drinks, cytotoxicity of Ervatamia leaves was examined.

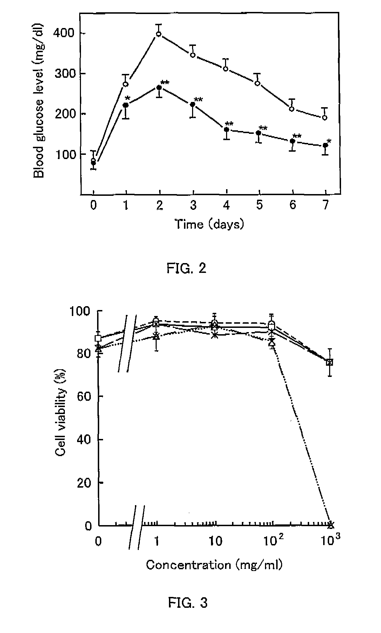

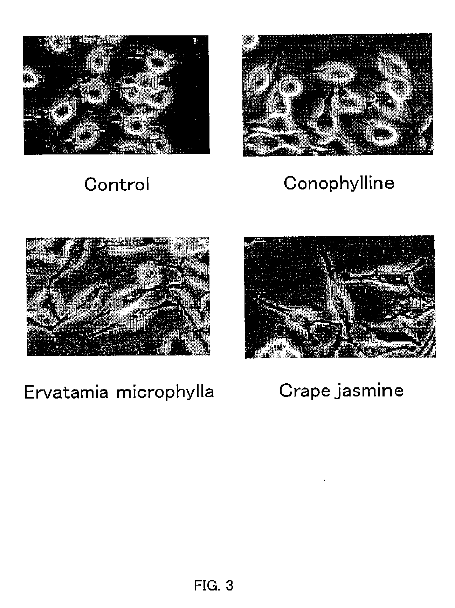

5.0 g of Ervatamia microphylla leaves was pulverized in a blender, methanol was applied as a solvent, and the mixture was stirred for 2 hours. An aqueous extract was prepared by extracting the active ingredient contained in the leaves into the solvent. Then, the aqueous extract was suction-filtered to remove solids, concentrated and dried to yield 962.7 mg of an extract. The extract obtained was dissolved in methanol and this methanol solution was chromatographed on a silica gel, eluting with acetone: toluene (1:8) and then the ratio of acetone was increased. Subsequently, the eluent was changed to methanol/chloroform (1:4) and then the ratio of methanol was increased (final proportion of methanol: 100%) to afford five fractions. The solutions of the five fractions were then concentrated and dried, and thus the components contained in each of the fractions were recovered. The components in each of the fractions were dissolved in methanol, and subsequently were added to the culture media containig K-ras-NRK cells (4 x 10⁵ cells) at concentrations of 1, 10, 100, and 1000 µg/ml. After culturing for 24 hours, the trypan blue exclusion test was performed to evaluate the cytotoxicity of each fraction from Ervatamia leaves. The results are shown in FIG. 3.

As shown in FIG. 3, it was revealed that, by treating K-ras-NRK cells at a high concentration (1000 µg/ml) of fraction 3 and fraction 4, remarlkable cell death occurs. This indicates that Ervatamia microphylla leaves have no cytotoxicity as long as their extract is used at low concentrations. It should be noted that no cytotoxicity was detected in the other fractions (1, 2, and 5)

### EXAMPLE 5

Conophylline is known to induce a morphological change of K-ras-NRK cells. Thus, it was examined whether an extract of Ervatamia microphylla leaves induces morphological changes of K-ras-NRK cells. It is also known that leaves of the Malaysian Tabernaemontana contains conophylline (Org. Biomol. Chem. 1: 1292-1297, 2003). Thus, it was examined that whether an extract of leaves of crape jasmine, the Japanese Tabernaemontana divaricata, also induces morphological changes in K-ras-NRK cells. An extract of each plant was prepared, in the same manner as described in Example 1, by extracting the active ingredient contained in the leaves with methanol, then removing solids by suction filtration, followed by concentring and drying , and dissolving the resulting powder into methanol.

500 µl of a suspension (2.0 x 10⁴ cells/ml per culture medium (DMEM containing 5% FBS, 0.6 g/l glutamine, 0.2 g/l kanamycin, 2.25 g/l NaHCO₃) ) of K-Ras-NRK cells was added to each well of 24-well plastic plates at 2.0 x 10⁴ cells/well, and the plates were incubated at 37°C under 5% CO₂. The next day, conophylline (final concentration: 0.1 µg/ml), an extract of Ervatamia microphylla leaves (final concentration: 10 µg/ml), and an extract of crape jasmine leaves (final concentration: 10 µg/ml) were added to the culture medium, which was incubated at 37°C under the condition of 5% CO₂ for three days. The cells cultured in the medium without addition of conophylline, an extract of Ervatamia microphylla leaves, nor an extract of crape jasmine leaves were used as the control. The results are shown in FIG.4.

As shown in FIG. 4, it was revealed that, like cells treated with conophylline, cells treated with an extract of Ervatamia microphylla leaves and those treated with an extract of crape jasmine leaves undergo a morphological change. This suggests that extracts of these leaves does not contain any inhibitor that inhibits the effect of conophylline. It has, therefore, been clarified that, by using these leaves, the same efficacy as that of conophylline can be obtained without the need of purifying conophylline.

### INDUSTRIAL APPLICABILITY

According to the present invention, dried products or extracts of leaves of Ervatamia microphylla or Tabernaemontana divaricata; health foods containing as an effective ingredient leaves of Ervatamia microphylla or Tabernaemontana divaricata of dried products of the leaves or extracts of the leaves or of dried products of the leaves; and methods for producing the same, for preventing or improving diabetes, can be provided.

## Claims

1. A health food for preventing or improving diabetes, comprising as an effective ingredient an Ervatamia microphylla leaf, a dried product of the leaf, or an extract of the leaf or of the dried product of the leaf.

2. The health food of claim 1, further comprising hepatocyte growth factor.

3. The health food of claim 1 or 2, further comprising nicotinamide.

4. A health food for lowering blood glucose levels, comprising as an effective ingredient an Ervatamia microphylla leaf, a dried product of the leaf, or an extract of the leaf or of the dried product of the leaf.

5. A method for producing a health food for preventing or improving diabetes, comprising using as a basic ingredient an Ervatamia microphylla leaf, a dried product of the leaf, or an extract of the leaf or of the dried product of the leaf.

6. A method for producing a health food for lowering blood glucose levels, comprising using as a basic ingredient an Ervatamia microphylla leaf, a dried product of the leaf, or an extract of the leaf or of the dried product of the leaf.

7. A dried product of an Ervatamia microphylla leaf.

8. An extract of an Ervatamia microphylla leaf or of a dried product of the leaf.

9. A health food for preventing or improving diabetes, comprising as an effective ingredient an Ervatamia microphylla leaf, a dried product of the leaf, or an extract of the leaf or of the dried product of the leaf.

10. The health food of claim 1, further comprising hepatocyte growth factor.

11. The health food according to claim 1 or 2 further comprising nicotinamide.

12. A health food for lowering blood glucose levels, comprising as an effective ingredient a Tabernaemontana divaricata leaf, a dried product of the leaf, or an extract of the leaf or of the dried product of the leaf.

13. A method for producing a health food for preventing or improving diabetes, comprising using as a basic ingredient a Tabernaemontana divaricata leaf, a dried product of the leaf, or an extract of the leaf or of the dried product of the leaf.

14. A method for producing a health food for lowering blood glucose levels, comprising using as a basic ingredient a Tabernaemontana divaricata leaf, a dried product of the leaf, or an extract of the leaf or of the dried product of the leaf.

15. A dried product of a Tabernaemontana divaricata leaf.

16. An extract of a Tabernaemontana divaricata leaf or of a dried product of the leaf.
